# EUROPEAN PATENT SPECIFICATION

(11) **EP 2 252 360 B1**
(45) Date of publication and mention of the grant of the patent: **16.05.2018**
(21) Application number: 09714311.9
(22) Date of filing: 26.02.2009
(51) Int. Cl.: A61M 29/02

(54) **BALLOON CATHETER WITH DURABLE TIP PORTION**
BALLONKATHETER MIT LANGLEBIGEM SPITZENABSCHNITT
CATHÉTER À BALLONNET AVEC PARTIE D EXTRÉMITÉ DURABLE

(30) Priority: 26.02.2008 US 31637 P
(43) Date of publication of application: 24.11.2010
(73) Proprietor: Boston Scientific Scimed, Inc., Maple Grove, MN 55311 (US)
(72) Inventor: GREGORICH, Daniel J., St. Louis Park Minnesota 55416 (US); GUNDALE, Ben, Plymouth Minnesota 55447 (US)
(74) Representative: Vossius & Partner Patentanwälte Rechtsanwälte mbB
(86) International application number: PCT/US2009/035350
(87) International publication number: WO 2009/108816

(56) References cited:
- WO-A-01/51114
- WO-A-89/02763
- WO-A-2007/130739

## Description

### Field of the Invention

This invention relates to the medical devices and more particularly to catheters, such as balloon catheters and/or stent delivery catheters.

### Background

Intravascular diseases are commonly treated by relatively non-invasive techniques such as percutaneous transluminal angioplasty (PTA) and percutaneous transluminal coronary angioplasty (PTCA). These therapeutic techniques are well known in the art and typically involve the use of a balloon catheter with a guidewire, possibly in combination with other intravascular devices such as stents. Some typical balloon catheters have an elongate shaft with a balloon attached proximate the distal end and a manifold attached to the proximal end. In use, some balloon catheters are advanced over a guidewire such that the balloon is positioned adjacent a restriction in a diseased vessel. The balloon is then inflated and the restriction in the vessel is opened. In some cases, balloon catheters are used in stent delivery/deployment procedures. A stent may be crimped or otherwise disposed about the balloon, the balloon and stent are navigated to a desired location within a vessel, and the balloon is then inflated to deploy the stent. In yet other embodiments, certain stent structures, such as self expanding stents, or the like, may be delivered using stent delivery catheters that do not include an expandable member and/or balloon construction.

Some basic types of intravascular catheters for use in such procedures include, for example, fixed-wire (FW) catheters, over-the-wire (OTW) catheters and single-operator-exchange (SOE) catheters. The general construction and use of FW, OTW and SOE catheters are all well known in the art. An example of an OTW catheter may be found in commonly assigned U.S. Pat. No. 5,047,045 to Arney et al. An example of an SOE balloon catheter is disclosed in commonly assigned U.S. Pat. No. 5,156,594 to Keith.
WO 2007/130739 discloses a catheter tip for attachment with an intravascular balloon catheter that is used within a body lumen. The catheter tip includes a body portion having a base that is attached to the balloon catheter.

A number of different catheter structures and assemblies are known, each having certain advantages and disadvantages. However, there is an ongoing need to provide alternative catheter structures and assemblies.

### Summary

The invention relates to alternative designs, materials and methods of manufacturing catheter structures and assemblies.
The invention is defined by the features of independent claim 1. Further preferred embodiments are defined in the dependent claims.
Embodiments of the invention relate to a catheter including an elongated shaft including distal portion and having a distal end. A durable polymeric distal tip structure is disposed on the distal end of the shaft, the distal tip structure including a distal ring portion configured to enhance the durability of the distal end of the catheter, and a proximal portion extending proximally from the distal ring portion and connecting the distal tip structure to the distal end of the catheter, wherein the proximal portion of the distal tip structure is more laterally flexible than the distal ring portion. The distal ring portion is made of a polymeric material that is harder than the materials of the distal portion of the elongated shaft and wherein at least a portion of the distal tip structure includes a radiopaque material. In some embodiments, distal tip structure is configured and/or disposed on the distal end of the shaft such that it may enhance resistance to deformation and/or flaring of the catheter. In some embodiments, the catheter is a balloon catheter and includes an expandable member affixed to the distal portion of the elongated shaft such that a section of the elongated shaft extends through at least a portion of the expandable member. In some other embodiments, the catheter configured for delivery of a self expanding stent, and may not include a balloon and/or expendable member. In some embodiments, the durable distal tip structure comprises a metal or metal alloy. In some embodiments, the distal ring portion and proximal portion of the durable distal tip structure comprise a single monolith of material.

Some embodiments relate to a durable distal tip structure for an intravascular catheter that may include a distal portion comprising a ring of durable material configured to provide a durable distal tip to the catheter, and a proximal portion extending proximally from the distal portion and configured for connecting the distal tip structure to the catheter, wherein the proximal portion of the distal tip structure is more laterally flexible than the distal portion. In some embodiments, the durable distal tip structure comprises a metal or metal alloy, and in some embodiments, the distal ring portion and proximal portion of the durable distal tip structure comprise a single monolith of material.

Some other embodiments relate to methods of making and using such catheter constructions and/or methods of making and using such durable distal tip structures.

Some other embodiments relate to methods of making a distal tip structure for an intravascular catheter, the method comprising providing an elongated tubular member of durable material; selectively removing portions of the material from the elongated tubular member to form a proximal portion extending proximally from a distal portion, the proximal portion being configured for connecting the distal tip structure to the catheter and the distal portion comprising a ring of durable material configured to provide a durable distal tip to the catheter, wherein the proximal portion of the distal tip structure is more laterally flexible than the distal portion.

The above summary of some embodiments is not intended to describe each disclosed embodiment or every implementation of the present invention. The Figures and Detailed Description which follow more particularly exemplify these embodiments.

### Brief Description of the Drawings

The invention may be more completely understood in consideration of the following detailed description of various embodiments of the invention in connection with the accompanying drawings, in which:
Figure 1 is a cross-sectional view of an example embodiment of a balloon catheter;
Figure 2 is an enlarged cross-sectional view the distal portion of the catheter of Figure 1;
Figure 3 is a perspective view of one example embodiment of a distal tip member, for example, as used in the catheter of Figure 1;
Figure 4 is a perspective view showing a mandrel, an inner tubular member, and a distal tip member arranged during an intermediate step during one example manufacturing method;
Figure 5 is a perspective view showing a mandrel, an inner tubular member, a distal tip member, and a balloon member arranged during an intermediate step during one example manufacturing method;
Figure 6 is a perspective view showing a mandrel, an inner tubular member, a distal tip member, and a balloon member arranged during a final step of manufacturing, prior to the removal of the mandrel;
Figure 7 is a perspective view of another example embodiment of a distal tip member;
Figure 8 is a perspective view of another example embodiment of a distal tip member;
Figure 9 is a perspective view of another example embodiment of a distal tip member;
Figure 10 is a perspective view of another example embodiment of a distal tip member;
Figure 11 is a perspective view of another example embodiment of a distal tip member;
Figure 12 is a perspective view of another example embodiment of a distal tip member;
Figure 13 is a perspective view of another example embodiment of a distal tip member; and
Figure 14 is a cross-sectional view of another example embodiment of a balloon catheter.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention.

### Detailed Description of Some Embodiments

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

Weight percent, percent by weight, wt%, wt-%, % by weight, and the like are synonyms that refer to the concentration of a substance as the weight of that substance divided by the weight of the composition and multiplied by 100.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description of some embodiments should be read with reference to the drawings, wherein like reference numerals indicate like elements throughout the several views. The drawings, which are not necessarily to scale, depict some example embodiments and are not intended to limit the scope of the invention. Those skilled in the art and others will recognize that many of the examples provided have suitable alternatives which may also be utilized.

As indicated above, the invention relates to alternative designs, materials and methods of manufacturing alternative catheter structures and assemblies. Several characteristics that may be desirable in intravascular catheters include pushability, trackability and crossability. Pushability refers to the ability to transmit force from the proximal end of the catheter to the distal end of the catheter. Trackability refers to the ability to navigate tortuous vasculature. Crossability refers to the ability to navigate the balloon catheter across narrow restrictions in the vasculature, such as stenosed vessels or fully and partially deployed stents.

Another characteristic that may be desirable in intravascular catheters is durability. Durability refers to the ability to better withstand the rigors of being handled and navigated within the tortuous anatomy without sustaining significant damage that may reduce performance characteristics. In many conventional catheter designs, generally soft and/or flexible polymer materials are used in constructing catheter tips, to provide for desired flexibility characteristics (e.g. trackability). However, the inventors have discovered that in some cases, and in certain circumstances, such soft polymer tip materials may have a lower level of durability, and can be susceptible to damage during handling and/or use. Catheter tip damage may occur, for example, when the catheter is navigated across narrow restrictions or occlusions in the vasculature, such as stenosed vessels or total chronic occlusions, or fully and partially deployed stents, or other rigors that may be encountered during handling or use. Such tip damage may reduce the desired performance characteristics of the catheter. The inventors have also discovered that during navigation in tortuous anatomy, in some cases, and in certain circumstances, such soft polymer tip materials may have tendency to deform (e.g. deform from an original, say circular cross-sectional shape, to more of an oval shape) and/or flair from their initial configuration, in particular when the tip is being navigated through a tight bend in the anatomy. Such deformation and/or flaring may reduce the desired performance characteristics and/or make the tip more susceptible to damage. For example, such deformation and/or flaring of the tip may make the catheter more susceptible to tip-catch and/or to hang-up in the anatomy or structures within the anatomy, which may be an undesirable performance characteristic and may lead to tip damage.

It would be desirable, therefore, to provide a catheter having improved durability, particularly near the distal tip. It would also be desirable to provide a catheter with increased tip durability, but while also allowing for other desirable characteristics to be maintained, such as flexibility, pushability, trackability and crossability. As such, at least some embodiments relate to alternative catheter designs and assemblies including structure related to increasing the durability of the catheter, particularly near the distal tip, and that may also allow for the maintenance of a certain degree of other desirable characteristics, such as flexibility, pushability, trackability and crossability. It would also be desirable to provide a catheter with enhanced resistance to deformation and/or flaring, in particular near the distal tip. As such, at least some embodiments relate to alternative catheter designs and assemblies including structure related to increasing the resistance to deformation and/or flaring. Some embodiments may include a structure or structures that achieve one or more, or possibly each of these desired properties.

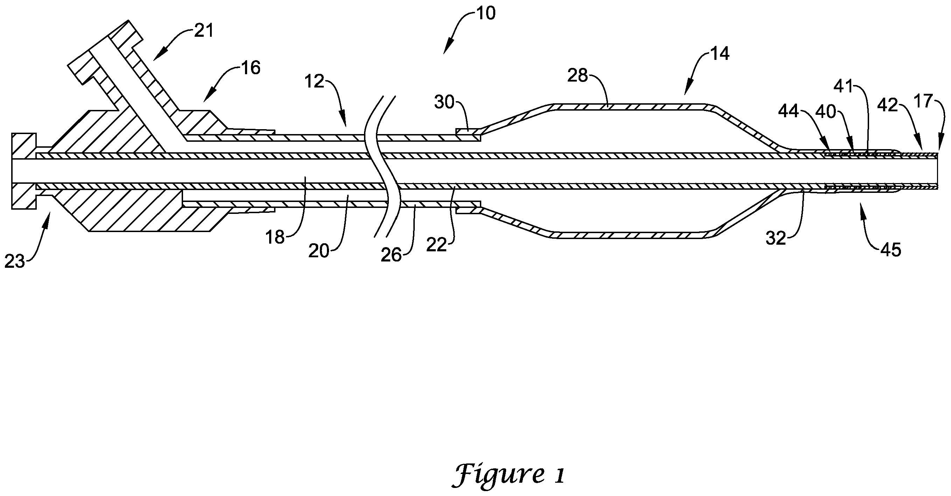

Referring now to the drawings, Figure 1 is a cross-sectional view of an over-the-wire (OTW) balloon catheter 10, which is representative of one example type of catheter that can incorporate at least certain aspects of the invention. Other intravascular catheter embodiments are additionally suitable without deviating from the scope of the invention. For example, some other suitable intravascular catheters may include fixed-wire (FW) catheters, single-operator-exchange (SOE) catheters, and the like. Some examples of OTW catheters are disclosed in commonly assigned U.S. Pat. No. 5,047,045 to Arney et al.,. Some examples of SOE balloon catheters are disclosed in commonly assigned U.S. Pat. No. 5,156,594 to Keith, Other catheter constructions, such as stent delivery catheters for self expanding stents, which do not necessarily include an expandable member and/or balloon, can also incorporate at least certain aspects of the invention

The balloon catheter 10 can include a shaft assembly 12 and an expandable assembly, such as a balloon assembly 14, connected proximate the distal end of shaft assembly 12. The shaft assembly 12 may have conventional dimensions and may be made of conventional materials suitable for intravascular navigation as in, for example, conventional angioplasty, stent deployment procedures, or the like. Some examples of balloon catheter constructions and materials are disclosed in U.S. Pat. Nos. 6,113,579; 6,623,504; and 6,761,703; and U.S. Pat. Publication Nos. 2006/0135979; 2005/0215950; 2005/0187536; 2004/0236276; 2004/0158256; 2003/0120207; 2005/0234499; and 2007/0005009.

In some embodiments, the catheter shaft 12 comprises at least two lumens extending within the catheter shaft 12. At least one lumen can be a device and/or guidewire lumen 18 that is adapted and/or configured to receive a guidewire or other such medical device. In some embodiments, the lumen 18 may extend the entire length of the catheter shaft 12 (e.g. over-the-wire catheter), or it may extend along a portion of the catheter shaft 12, wherein it exits the catheter shaft 12 at the distal end 17 (e.g. single operator exchange catheter). The catheter shaft 12 can also include one or more additional lumens, for example, an inflation lumen 20. The inflation lumen 20, for example, may allow for fluid communication between an inflation source and the balloon assembly 14. In general, the proximal end of the inflation lumen 20 can be put into fluid communication with an inflation source while the distal end of the inflation lumen 20 is in fluid communication with the interior of the balloon assembly 14. The shaft assembly 12 may be a multiple lumen design or a coaxial design as shown.

In the co-axial design shown, the shaft assembly 12 can include an inner tubular member 22 and an outer tubular member 26. The inner tubular member 22 defines the guidewire lumen 18, and the outer tubular member 26 is co-axially disposed about the inner tubular member 22 to define the annular inflation lumen 20 there between.

In some embodiments, a manifold assembly 16 may be connected to the proximal end 19 of the shaft assembly 12. An example of a conventional OTW-type manifold assembly 16 is shown, but other types of manifolds are contemplated. In the example shown, one branch 21 of this manifold assembly 16 may be adapted and/or configured to connect an inflation source to the inflation lumen 20, and may be used to inflate and deflate an inflatable member 28. Another branch 23 of this manifold assembly 16 may connect to the guidewire lumen 18, and may be used for insertion of a guidewire or other such device into the lumen 18.

The balloon assembly 14 can include an expandable balloon portion 28, a proximal balloon waist 30 and a distal balloon waist 32. The proximal balloon waist 30 connects the balloon assembly 14 to the outer tubular member 26 near its distal end using suitable attachment means, for example, an adhesive, a thermal bond, a mechanical bond, or the like. The distal balloon waist 32 similarly connects the balloon assembly 14 to the inner tubular member 22 near its distal end using suitable attachment means, for example, an adhesive, a thermal bond, a mechanical bond, or the like. The inner tubular member 22 extends through at least a portion of the expandable balloon portion 28 in a generally coaxial manner. In certain embodiments, the distal balloon waist 32 is only connected to the inner tubular member 22 which extends beyond the distal balloon waist 32. In alternative embodiments, the distal balloon waist 32 can be connected to the inner tubular member 22 and to a distal tip member 41 as will be discussed further below. The catheter includes a distal tip portion 45 which includes the structure that extends distally of the inflatable balloon portion 28.

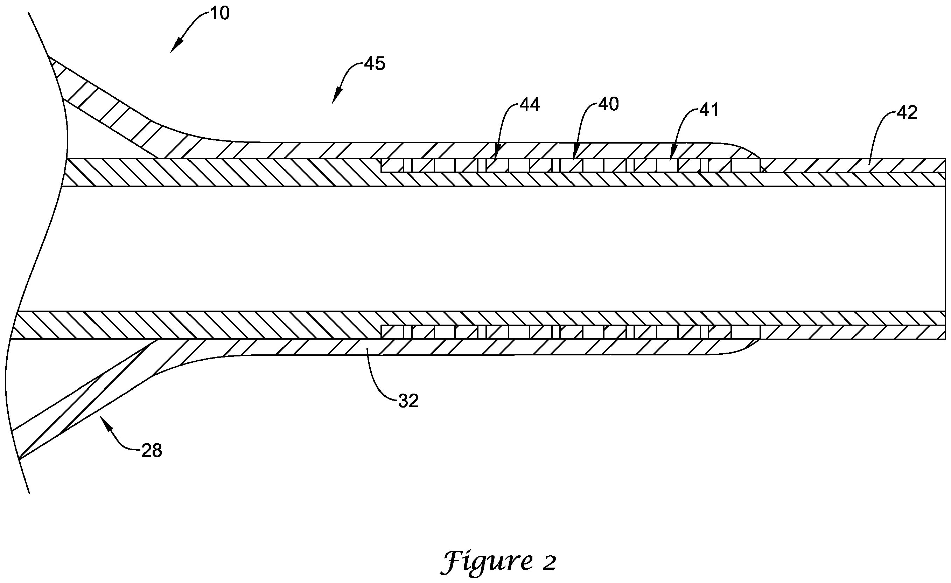

The catheter 10 includes a distal tip structure 40 disposed in the distal tip portion 45 at the distal end of the shaft 12. Referring now to Figure 2, which is a blow-up of the distal tip portion 45 of the catheter 10, the distal tip structure 40 in this embodiment includes a distal tip member 41 that includes a distal portion 42 and a proximal portion 44.

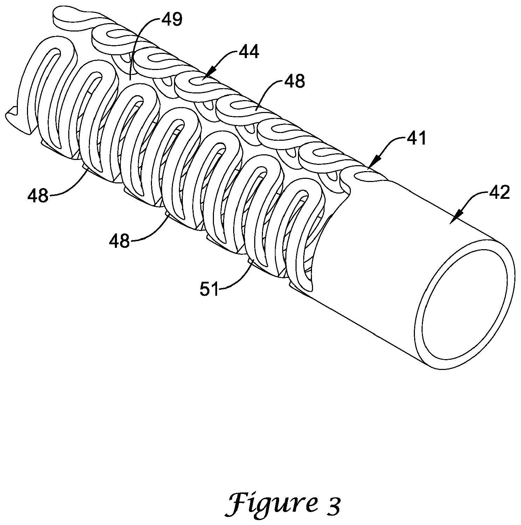

Figure 3 shows a perspective view of the example distal tip member 41 removed from the other structure of the catheter 10. The distal portion 42 may be configured as an annular, ring like, and/or tubular structure that can be configured to be disposed at the distal end of the catheter shaft 12, and may be adapted and/or configured to enhance the durability of the distal tip of the catheter 10. In some embodiments, as in the one shown, the distal portion 42 may be a generally solid ring and/or tubular member without any slots or apertures defined therein. The proximal portion 44 may be connected to, and extend proximally from, the distal portion 42, and may be configured to aid in connecting the distal tip member 41 to the catheter. The proximal portion 44 may also be configured to allow for a certain degree of increased lateral flexibility along its length relative to the distal portion 42. In some embodiments, as in the one shown, the proximal portion 44 may include structure 48, such as one or more struts, legs, coils and/or or tubular member having one or more slots, openings, grooves, voids, or the like defined therein, or other such structure that may aid in providing a desired degree of lateral flexibility, as well as providing structure for attaching the distal tip member 41 to the catheter 10. In some embodiments, the distal tip member 41 may be characterized in that the distal portion 42 provides a structure for enhancing the durability of the tip of the catheter construction, and the proximal portion 44 provides a structure for attaching the distal tip member 41 to the catheter construction while also allowing for a certain degree of desirable flexibility characteristics to be maintained. A discussion of some examples of particular configurations for various distal tip structures, including that shown in Figure 3, will be discussed in more detail below.

At least the distal ring portion 42, and in some embodiment the entire distal tip structure 40, is made of a material that is more durable and/or stronger and/or harder and/or has a higher modulus of elasticity than the material of the other structures in the tip portion 45. For example, the distal tip structure 40 is made of a material that is more durable and/or stronger and/or harder and/or has a higher modulus of elasticity relative to the material of the inner tubular member 22, the distal balloon waist 32, and/or other structures that may be used in the tip portion 45. Typically the material chosen is more durable and/or stronger and/or harder than typical materials used in conventional balloon catheter tip constructions. The use of such a durable material allows for the distal portion 42 to provide a more durable tip to the catheter shaft 12, and reduce the likelihood of significant tip damage during normal use and/or handling. Such a construction may also aid in providing enhanced crossability for the catheter, particularly for stenosed lesions and/or occlusions, such as total chronic occlusions. Additionally, such a construction may also aid in enhancing resistance to deformation and/or flaring of the distal tip portion 45 of the catheter. For example, because the distal tip structure 40 and/or the distal portion 42 may be made of a material that is more durable and/or stronger and/or harder and/or has a higher modulus of elasticity relative to the material of the inner tubular member 22, the distal balloon waist 32, and/or other structures that may be used in the tip portion 45, and due to its positioning within the tip portion 45, it may reduce and/or prevent flaring and/or deformation of the tip portion 45. Such characteristics may be true of any of the embodiments disclosed herein, and others.

The distal ring portion may comprise between 10 and 50%, between 15 and 40% or between 20 and 30% of the entire length of the distal tip structure.

Some examples of suitable durable materials for use in the distal portion 42 and/or distal tip structure 40 may include metal, metal alloy, ceramic, polymer with increased durability, strength, hardness and/or modulus relative to the other polymers used in the catheter tip construction, or composites or combinations of any of these materials. Due to the increased durability, in many embodiments, metal and/or metal alloys may be advantageous used.

Some examples of suitable metals and/or alloys can include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; platinum-chromium alloys; titanium; combinations thereof; and the like; or any other suitable material. In at least some embodiments, portions or all of the distal tip structure 40 may be made of, doped with, or otherwise include a radiopaque material. Radiopaque materials are understood to be materials capable of producing a relatively bright image on a fluoroscopy screen or another imaging technique during a medical procedure. This relatively bright image may aid the user of the catheter in determining its location. Some examples of radiopaque materials can include, but are not limited to, gold, platinum, palladium, tantalum, tungsten alloy, polymer material loaded with a radiopaque filler, and the like. Additionally, other radiopaque structures, such as marker bands, coils, or others, may also be incorporated into the design of catheter to achieve the desired result.

Some examples of suitable durable polymers that may be used in the distal portion 42 and/or distal tip structure 40 may include high modulus polymers (e.g. high durability polymers), such as polycarbonate, polyimid, poly-ether-ether-ketone (PEEK), or the like, or other suitable high modulus/durable polymers. Again, these polymers are doped with and/or include radiopaque materials.

Some examples of suitable composites can include glass-polymer composites, carbon-polymer composites, silica-polymer composites, metal-polymer composites, and the like, or others. Again, these composites may be doped with and/or include radiopaque materials.

In some embodiments, the distal tip member 41 can be constructed from a single and/or monolithic tubular member, such as a hypotube, or other tubular monolith of material, and the desired structure of the distal tip member 41 may be formed by the selective removal of material from such a structure. For example, a single tubular monolith of material may be provided, and through techniques such as laser cutting, micro-machining, etching, grinding, or the like, material can be selectively removed from the tubular member to form the desired structure, for example, in the proximal portion 44. It is also contemplated, however, that the distal and proximal portions 42/44 of the distal tip member 41 may be separate components that are thereafter attached to one another using suitable attachment techniques to create the distal tip member 41. However, for simplicity and ease of construction, the use of a single monolith and selective removal of material to create the desired structure may be advantageous.

As mentioned above, the proximal portion 44 may include structure that allows for good connection of the distal tip member 41 to the catheter, while also potentially allowing for a certain degree of lateral flexibility characteristics to be maintained. For example, in the embodiment shown in Figure 3, the proximal portion 44 includes a plurality of legs and/or struts 48 extending proximally from the distal portion 42, and the struts 48 are separated by gaps and/or slots 49 where material has been removed from the tubular member to form the struts 60. In this embodiment, there are three struts 48 having a series of aggressive and/or tight pitch undulations or curves 51 that may allow for a good connection with the catheter and also may allow for desired flexibility characteristics. It should be understood that in other embodiments, more or fewer such legs and/or struts 48 may be used and/or defined, as desired. As can be appreciated, the legs and/or struts 48 may resemble, for example, the struts of a stent. Some example stent structures and/or patterns that may be used in defining the structure of the proximal portion 44 can include those disclosed in U.S. Pat. Nos. 7,060,088; 6,955,686; and 7,029,493 Those of skill in the art and others will recognize that a wide variety of other structures may be used in defining the proximal portion 44, depending upon the desired properties and/or characteristics. In many embodiments, the particular structure used may provide for a good connection of the distal tip member 41 to the catheter while also providing for enhanced lateral flexibility of the proximal portion 44 relative to the distal portion 42.

The distal tip member 41 may be sized as necessary to provide the desired characteristics. By way of example only, in some embodiments, the distal tip member 41 may be formed from a generally tubular member having a wall thickness in the range of about 2,54mm to 0,05mm (0,1 to about 2 thousands of an inch). As such, the distal ring portion 42 and/or the proximal portion 44 may also have a similar wall thickness. In some embodiments, the distal ring portion 42 may have a length in the range of about 12,7mm to 0,2mm (0,5 to about 4 thousands of an inch). In some embodiments, the proximal portion 44 may have a length in the range of about 0.5 to about 10 mm. Again, it should be understood that these dimensions are given by way of example only, and that other dimensions may be used, depending upon the desired characteristics of the device.

The distal tip member 40 may be secured to the catheter shaft using any suitable attachment technique. In the embodiment shown in Figures 1 and 2, the distal tip member 41 is disposed annularly about the distal end of the inner tubular member 22, and within a portion of the distal balloon waist 32. As such, at least a portion of the distal tip member 41 may be characterized as being disposed, sandwiched, and/or encased between a portion of the inner tubular member 22 and a portion of the balloon waist 32. One example method of making such a construction can generally include attaching the distal tip member 40 using the same technique and/or material that is used to attach the distal balloon waist 32 to the inner tubular member 22.

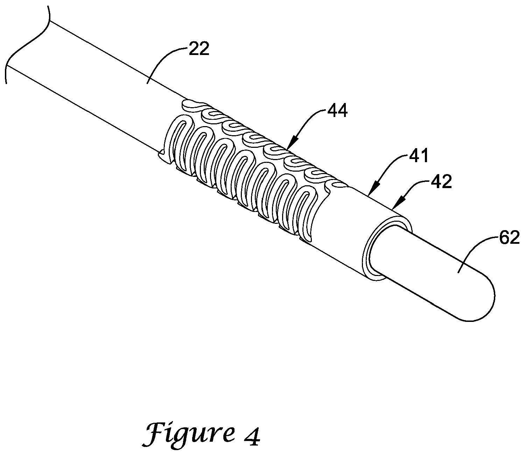

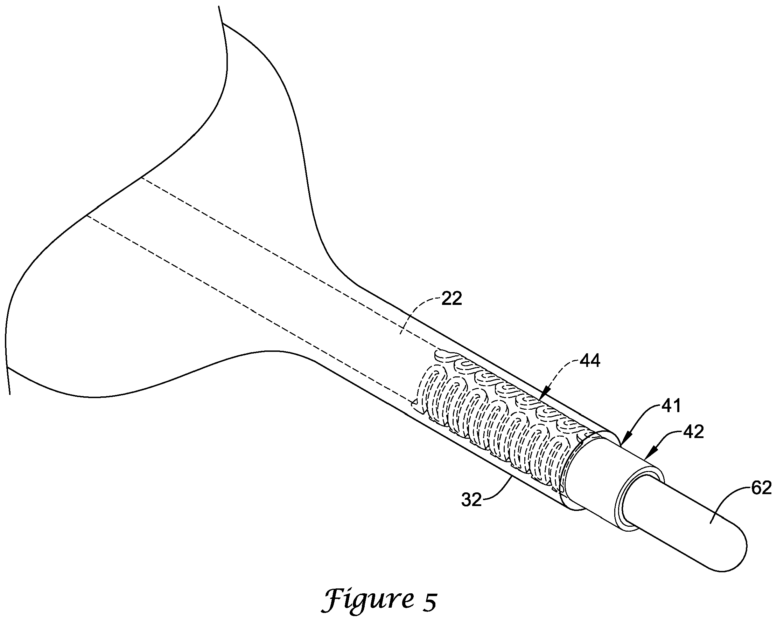

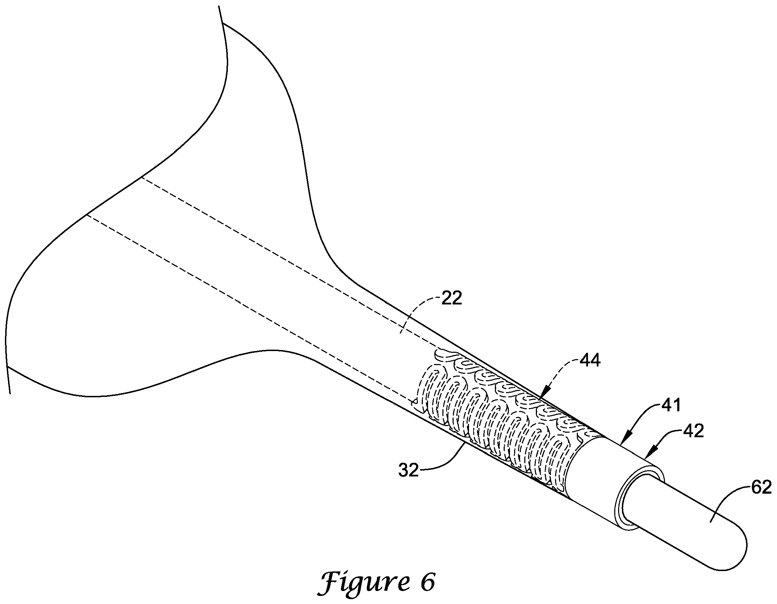

For example, Figures 4 through 6 illustrate one example of such a method. In Figure 4, the inner tubular member 22 is shown disposed about a mandrel 62, and the distal tip member 41 is disposed on the distal end of the inner tubular member 22. At this point, the outer tubular member (not shown) may also be threaded about the proximal portion of the inner tubular member. As shown in Figure 5, the balloon assembly 14 is threaded about the inner tubular member 22 and the distal tip member 41 such that a portion of the distal balloon waist 32 extends about and/or overlaps with a portion of the distal tip member 41. In some embodiments, the distal balloon waist 32 may extend about and/or overlap with the entire distal tip member 41, or may overlap with only the proximal portion 44 of the distal tip member 41, or possibly only a portion of the proximal portion 44 of the distal tip member 41. In the embodiment shown, the distal balloon waist 32 extends about and/or overlaps with the entire proximal portion 44 of the distal tip member 41. While not shown, the proximal balloon waist 30 may be disposed about the distal end of the outer tubular member 26. The distal waist 32 may then be secured to the inner tubular member 22 and to the distal tip member 41 using a suitable attachment technique. In some embodiments, the distal waist 32 may be heated and/or welded such that the material of the distal waist 32 flows and/or reflows onto the surface of the inner tubular member 22 and onto the surfaces and/or within the structure defined in the proximal portion 44 of the distal tip member 41, thereby attaching the entire construction together, as shown in Figure 6. If necessary, further assembling of the shaft 12, or portions and/or components to create the catheter 10, may be performed. For example, the proximal balloon waist 30 may be attached to the outer tubular member 26, and the manifold 16 may be attached to the proximal end of the shaft 12.

In other embodiments, the distal tip member 40 may be secured using other suitable attachment techniques. For example, instead of using the distal balloon waist 32 and/or balloon material to attach the distal tip member 41 to the inner tubular member 22, a separate polymer layer and/or material may be similarly used. Additionally, other attachment techniques, such as adhesive bonding, thermal bonding, shrink bonding, mechanical bonding, or the like, can be used to attach the distal tip member 41 to the distal end of the shaft 12. In yet other embodiments, techniques such as extrusion, co-extrusion, interrupted layer co-extrusion (ILC), molding, casting, forming, of the like, may be used to construct the inner tubular member and/or tip portion 45, and the distal tip member 41 could be incorporated into and/or attached during such construction techniques.

The distal tip member 41 can be disposed at any desired location within the tip of the catheter, depending upon the desired properties of the catheter. In some embodiments, the distal portion 42 may extend distally of any other structure in the catheter 10, and act as the as the durable leading edge and/or distal tip of the catheter 10. However, in other embodiments, the distal portion 42 may be co-terminus with one or more of the other structures at the distal end of the shaft 12 and provide a durable leading structure. For example, the distal end of the inner tubular member 22 and/or the distal balloon waist 32 may be coterminous with the distal end of the distal portion 42. In yet other embodiments, the distal end of the distal portion 42 may be slightly proximal of the distal end of other structures of the catheter 10, such as the distal end of the inner tubular member 22 and/or the distal balloon waist 32, but may still provide for enhanced durability due to its presence adjacent the distal tip.

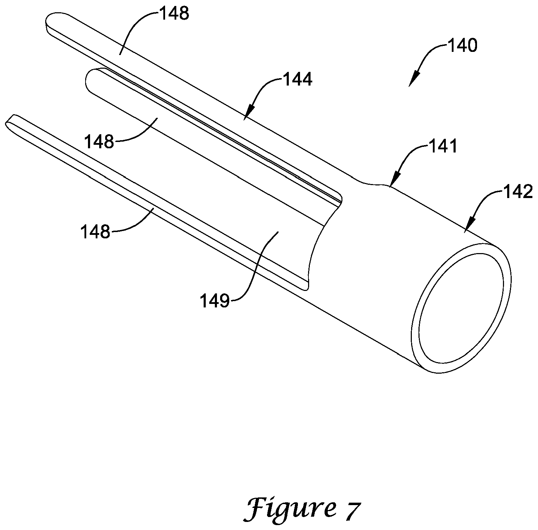

Refer now to Figure 7, which shows another alternative embodiment of a distal tip structure 140. In this embodiment, the distal tip structure 140 includes a distal tip member 141 having a distal portion 142 and a proximal portion 144. The distal portion 142 may again be configured as an annular, ring like, and/or tubular structure that can be configured to be disposed at the distal end of the catheter shaft 12, and may be adapted and/or configured to enhance the durability of the distal tip of the catheter 10. In some embodiments, as in the one shown, the distal portion 142 may be a generally solid ring and/or tubular member without any slots or apertures defined therein. The proximal portion 144 may be connected to, and extend proximally from, the distal portion 142, and may be configured to aid in connecting the distal tip member 141 to the catheter. The proximal portion 144 may also be configured to allow for a certain degree of increased lateral flexibility along its length relative to the distal portion 142. In the embodiment shown, the proximal portion 144 includes a plurality of legs and/or struts 148 extending proximally from the distal portion 142, and the struts 148 are separated by gaps and/or slots 149 where material has been removed from the tubular member to form the struts 148. In this embodiment, there are three struts 48 having a generally straight longitudinal configuration, and that may allow for a good connection with the catheter and also may allow for desired flexibility characteristics. It should be understood that in other embodiments, more or fewer such legs and/or struts 148 may be used and/or defined, as desired.

The distal tip member 141 may be manufactured, include materials, be sized, and be incorporated into a catheter construction similarly to that discussed above regarding the distal tip structure 40. For example, the distal tip member 141 can be made of and/or include durable materials as discussed above, may be constructed from a single and/or monolithic tubular member, as discussed above, may be attached and/or incorporated into the catheter shaft construction as discussed above.

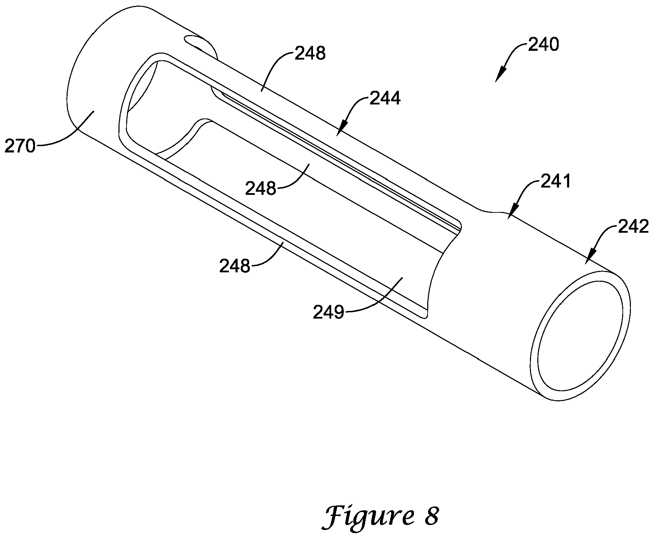

Refer now to Figure 8, which shows another alternative embodiment of a distal tip structure 240. In this embodiment, the distal tip structure 240 includes a distal tip member 241 having a distal portion 242 and a proximal portion 244. The distal portion 242 may again be configured as an annular, ring like, and/or tubular structure that can be configured to be disposed at the distal end of the catheter shaft 12, and may be adapted and/or configured to enhance the durability of the distal tip of the catheter 10. In some embodiments, as in the one shown, the distal portion 242 may be a generally solid ring and/or tubular member without any slots or apertures defined therein. The proximal portion 244 may be connected to, and extend proximally from, the distal portion 242, may be configured to aid in connecting the distal tip member 241 to the catheter 10. The proximal portion 244 may also be configured to allow for a certain degree of increased lateral flexibility along its length relative to the distal portion 242.

In the embodiment shown in Figure 8, similar to the embodiment shown in Figure 7, the proximal portion 244 includes a plurality of legs and/or struts 248 extending proximally from the distal portion 242, and the struts 248 are separated by gaps and/or slots 249 where material has been removed from the tubular member to form the struts 248. In this embodiment, there are three struts 248 having a generally straight longitudinal configuration, and that may allow for a good connection with the catheter and also may allow for desired flexibility characteristics. It should be understood that in other embodiments, more or fewer such legs and/or struts 248 may be used and/or defined, as desired. This embodiment, however, also includes a proximal annular and/or ring like structure 270 disposed at the proximal end of the proximal portion 244. As such, the struts 248 may be characterized as being intermediate structures between the distal and proximal ring structures 242/270. The proximal ring structure 270 may function to aid in making good connection of the distal tip member 241 to the catheter shaft. For example, the presence of the proximal ring structure 270 may allow for the creation of an enhanced mechanical interlock with the material of the distal balloon waist 32 when the balloon waist is flowed onto the outer surface of the inner tubular member during attachment.

The distal tip member 241 may be manufactured, include materials, be sized, and be incorporated into a catheter construction similarly to that discussed above regarding the distal tip structure 40. For example, the distal tip member 241 can be made of and/or include durable materials as discussed above, may be constructed from a single and/or monolithic tubular member, as discussed above, may be attached and/or incorporated into the catheter shaft construction as discussed above.

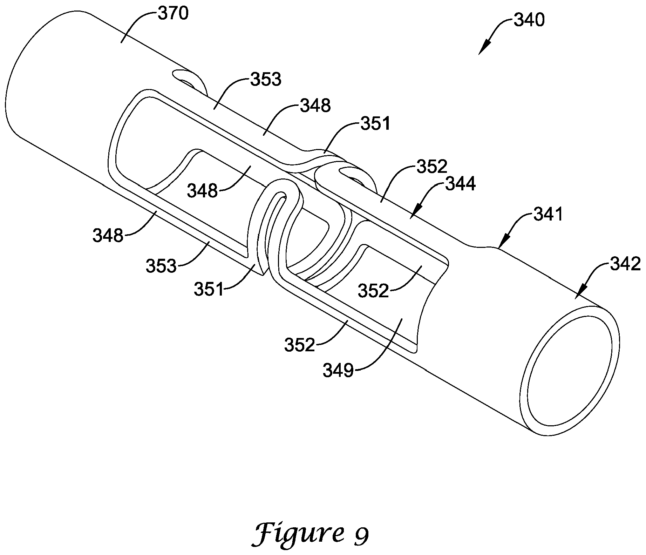

Refer now to Figure 9, which shows another alternative embodiment of a distal tip structure 340. In this embodiment, the distal tip structure 340 includes a distal tip member 341 having a distal portion 342 and a proximal portion 344. The distal portion 342 may again be configured as an annular, ring like, and/or tubular structure that can be configured to be disposed at the distal end of the catheter shaft 12, and may be adapted and/or configured to enhance the durability of the distal tip of the catheter 10. In some embodiments, as in the one shown, the distal portion 342 may be a generally solid ring and/or tubular member without any slots or apertures defined therein. The proximal portion 344 may be connected to, and extend proximally from, the distal portion 342, may be configured to aid in connecting the distal tip member 341 to the catheter 10. The proximal portion 344 may also be configured to allow for a certain degree of increased lateral flexibility along its length relative to the distal portion 342.

In the embodiment shown in Figure 9, similar to the embodiment shown in Figure 8, the proximal portion 344 includes a plurality of legs and/or struts 348 extending proximally from the distal portion 342, and the struts 348 are separated by gaps and/or slots 349 where material has been removed from the tubular member to form the struts 348. In this embodiment, there are three struts 348 that may allow for a good connection with the catheter and also may allow for desired flexibility characteristics. It should be understood that in other embodiments, more or fewer such legs and/or struts 348 may be used and/or defined, as desired. This embodiment also includes a proximal annular and/or ring like structure 370 disposed at the proximal end of the proximal portion 344. As such, the struts 348 may be characterized as being intermediate structures between the distal and proximal ring structures 342/370. The proximal ring structure 370 may function to aid in making good connection of the distal tip member 341 to the catheter shaft. For example, the presence of the proximal ring structure 370 may allow for the creation of an enhanced mechanical interlock with the material of the distal balloon waist 32 when the balloon waist is flowed onto the outer surface of the inner tubular member 22 during attachment.

In this embodiments, however, rather than the struts 349 including the generally straight longitudinal configuration along their entire length as in Figure 8, one or more of the struts 349 each include a curved and/or undulating portion 351 disposed between two straight portions 352 and 353. Such a configuration may aid in enhancing the lateral flexibility of at least a portion of the distal tip member 341, and may also aid in providing an enhanced mechanical interlock when the distal tip member 341 is attached to the catheter 10. Such undulations and/or curves may resemble those discussed above regarding the embodiment shown in Figure 3.

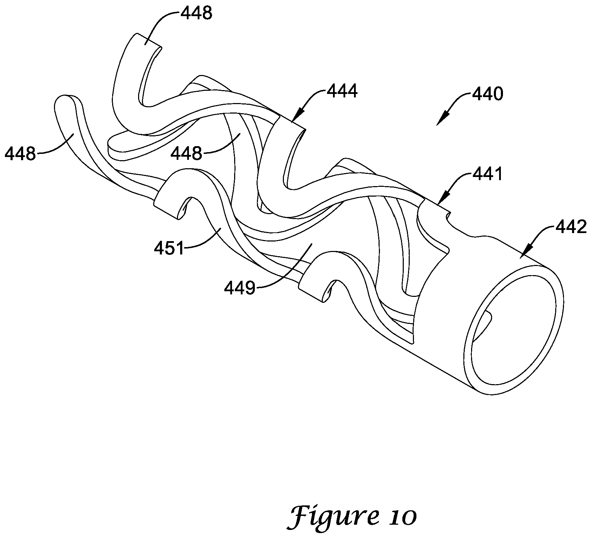

Refer now to Figure 10, which shows another alternative embodiment of a distal tip structure 440 which is similar in many respects to the embodiment shown in Figure 3. In this embodiment, the distal tip structure 440 again includes a distal tip member 441 having a distal portion 442 and a proximal portion 444. The distal portion 442 may again be configured as an annular, ring like, and/or tubular structure that can be configured to be disposed at the distal end of the catheter shaft 12, and may be adapted and/or configured to enhance the durability of the distal tip of the catheter 10. In some embodiments, as in the one shown, the distal portion 442 may be a generally solid ring and/or tubular member without any slots or apertures defined therein. The proximal portion 444 may be connected to, and extend proximally from, the distal portion 442, and may be configured to aid in connecting the distal tip member 441 to the catheter. The proximal portion 444 may also be configured to allow for a certain degree of increased lateral flexibility along its length relative to the distal portion 442. In the embodiment shown, the proximal portion 444 includes a plurality of legs and/or struts 448 extending proximally from the distal portion 442, and the struts 448 are separated by gaps and/or slots 449 where material has been removed from the tubular member to form the struts 448. In this embodiment, there are three struts 448 having a series of undulations and/or curved portions 451, somewhat similar to that shown and described in the embodiment of Figure 3. However, in this embodiment, the curves and/or undulations 451 are of a somewhat looser configuration (not as tightly pitched) and may be somewhat continuously curved such that there are relatively few straight portions. As such, the struts 448 may provide for different flexibility characteristics relative to the embodiment shown in Figure 3. Such a configuration may allow for a good connection with the catheter and also may allow for desired flexibility characteristics. It should be understood that in other embodiments, more or fewer such legs and/or struts 448 may be used and/or defined, as desired.

The distal tip member 441 may be manufactured, include materials, be sized, and be incorporated into a catheter construction similarly to that discussed above regarding the distal tip structure 40. For example, the distal tip member 441 can be made of and/or include durable materials as discussed above, may be constructed from a single and/or monolithic tubular member, as discussed above, may be attached and/or incorporated into the catheter shaft construction as discussed above.

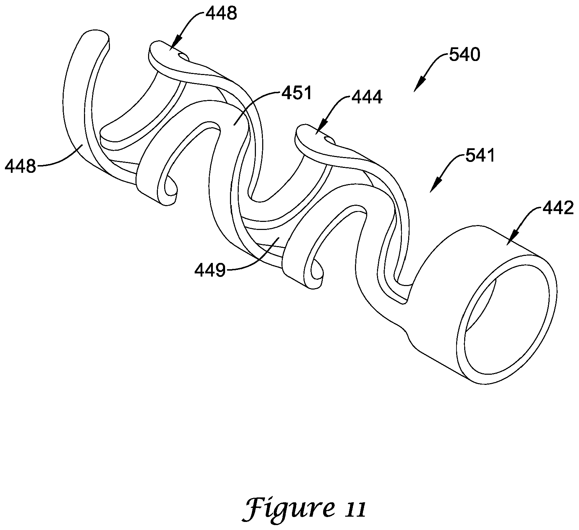

Refer now to Figure 11, which shows another alternative embodiment of a distal tip structure 540 including a distal tip member 541 which is similar in many respects to the embodiment 441 shown in Figure 10, wherein like reference number indicate similar structure. However, in this embodiment, there are two struts 448 rather than three. Again, the distal tip member 541 may be manufactured, include materials, be sized, and be incorporated into a catheter construction similarly to that discussed above regarding the distal tip structure 40. For example, the distal tip member 541 can be made of and/or include durable materials as discussed above, may be constructed from a single and/or monolithic tubular member, as discussed above, may be attached and/or incorporated into the catheter shaft construction as discussed above.

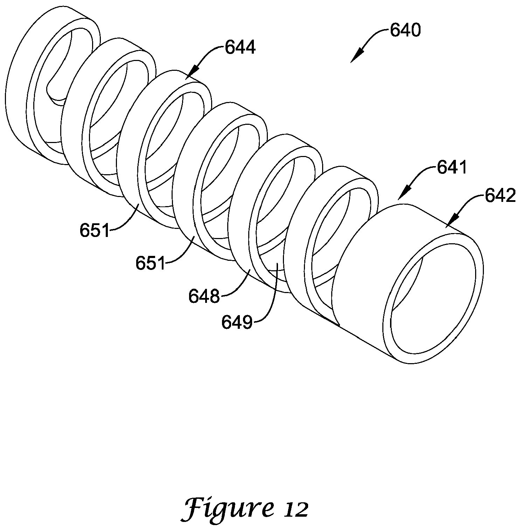

Refer now to Figure 12, which shows another alternative embodiment of a distal tip structure 640 which may be similar in many respects to those discussed above, but including different structure in the proximal portion 644. In this embodiment, the distal tip structure 640 again includes a distal tip member 641 having a distal portion 642 and a proximal portion 644. The distal portion 642 may again be configured as an annular, ring like, and/or tubular structure that can be configured to be disposed at the distal end of the catheter shaft 12, and may be adapted and/or configured to enhance the durability of the distal tip of the catheter 10. In some embodiments, as in the one shown, the distal portion 642 may be a generally solid ring and/or tubular member without any slots or apertures defined therein.

The proximal portion 644 may be connected to, and extend proximally from, the distal portion 642, and may be configured to aid in connecting the distal tip member 641 to the catheter. The proximal portion 644 may also be configured to allow for a certain degree of increased lateral flexibility along its length relative to the distal portion 642. In the embodiment shown, the proximal portion 644 includes a helical and/or coil like structure 648 extending proximally from the distal portion 442, and the turns of the helical structure 668 are separated by a gap and/or slots 649 where material has been removed from the tubular member to form the helical structure 468. In this embodiment, there is a single helical structure 668 defining a series turns 651. The helical structure 468 may be configured to achieve the desired properties of the proximal portion 644. For example, in some embodiments, the turns of the coil 668 may be tightly pitched such that each successive turn may be touching and/or almost touching the last turn. In other embodiments, as shown, the pitch may be more open, such that the gap and/or space between the turns is more open. In yet other embodiments, a combination of open and closed pitches may be used. As such, different pitch configurations may provide for a variety of different flexibility characteristics. Such configurations may allow for a good connection with the catheter and also may allow for desired flexibility characteristics. It should be understood that in other embodiments, more than one helical structure may be used. For example, two, three, or more helical structures may extend proximally from the distal portion 642 and define the proximal portion 644.

The distal tip member 641 may be manufactured, include materials, be sized, and be incorporated into a catheter construction similarly to that discussed above regarding the distal tip structure 40. For example, the distal tip member 641 can be made of and/or include durable materials as discussed above, may be constructed from a single and/or monolithic tubular member, as discussed above, may be attached and/or incorporated into the catheter shaft construction as discussed above.

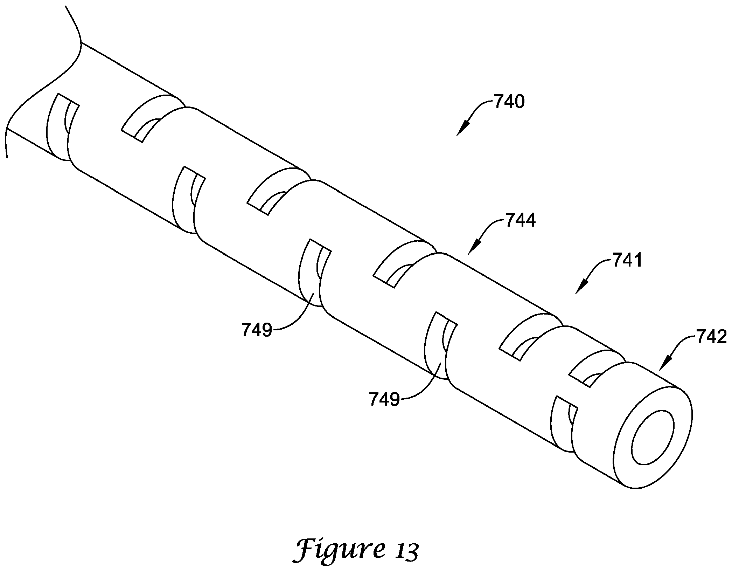

Refer now to Figure 13, which shows another alternative embodiment of a distal tip structure 740 which may be similar in many respects to those discussed above, but includes different structure in the proximal portion 744. In this embodiment, the distal tip structure 740 again includes a distal tip member 741 having a distal portion 742 and a proximal portion 744. The distal portion 742 may again be configured as an annular, ring like, and/or tubular structure that can be configured to be disposed at the distal end of the catheter shaft 12, and may be adapted and/or configured to enhance the durability of the distal tip of the catheter 10. In some embodiments, as in the one shown, the distal portion 742 may be a generally solid ring and/or tubular member without any slots or apertures defined therein.

The proximal portion 744 may be connected to, and extend proximally from, the distal portion 742, and may be configured to allow for a certain degree of increased lateral flexibility along its length relative to the distal portion 742. The proximal portion 744 may also include structure that may aid in connecting the distal tip member 741 to the catheter. In the embodiment shown, the proximal portion 744 includes an elongated tubular section including a plurality of slots and/or grooves 749 cut and/or formed therein where material has been removed from the tubular member. The slots 749 may be disposed in any pattern necessary to achieve the desired flexibility characteristics. For example, the size, density, shape and/or other characteristics of the slots may be used to achieve desired results. As such, various embodiments of arrangements and configurations of slots 749 are contemplated. In some embodiments, at least some, if not all of slots 749 are disposed at the same or a similar angle with respect to the longitudinal axis of the tubular distal tip member 741. As shown, slots 749 can be disposed at an angle that is perpendicular, or substantially perpendicular, and/or can be characterized as being disposed in a plane that is normal to the longitudinal axis of the tubular distal tip member 741. However, in other embodiments, slots 749 can be disposed at an angle that is not perpendicular, and/or can be characterized as being disposed in a plane that is not normal to the longitudinal axis of the tubular distal tip member 741. Additionally, a group of one or more slots 741 may be disposed at different angles relative to another group of one or more slots 741. The distribution and/or configuration of slots 741 can also include, to the extent applicable, any of those disclosed in U.S. Pat. Publication No. 2004/0181174. Some example embodiments of appropriate micromachining methods and other cutting methods, and structures for tubular members including slots and medical devices including tubular members are disclosed in U.S. Pat. Publication Nos. 2003/0069522 and 2004/0181174; and U.S. Pat. Nos. 6,766,720; and 6,579,246. Some example embodiments of etching processes are described in U.S. Pat. No. 5,106,455.

The distal tip member 741 may be manufactured, include materials, be sized, and be incorporated into a catheter construction similarly to that discussed above regarding the distal tip structure 40. For example, the distal tip member 741 can be made of and/or include durable materials as discussed above, may be constructed from a single and/or monolithic tubular member, as discussed above, may be attached and/or incorporated into the catheter shaft construction as discussed above.

As shown in the embodiments above, in many cases, the distal tip member (e.g. 41, 141, 241, 341, 441, 541, 641, 741) may be sized such that the proximal portion (e.g. 44, 144, 244, 344, 444, 544, 644, 744) ends distally of the expandable balloon portion 28. As such, the distal tip member may be disposed primarily in the distal portion 45 of the catheter. However, in some other embodiments, it is contemplated that the proximal portion of the distal tip member may extend further proximally, for example, proximally through the balloon, and/or possibly into the shaft proximal of the balloon. In some cases, it is contemplated that the proximal portion of the distal tip member may extend proximally to the proximal end of the shaft. In some such configurations, the proximal portion of the distal tip member may be characterized as making up and/or being part of the inner tubular member 22 structure.

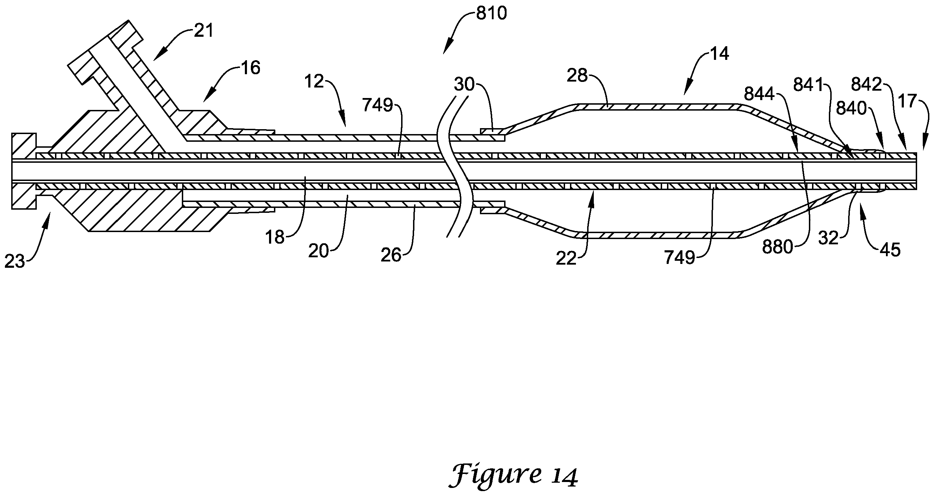

For example, refer now to Figure 14, which shows an alternative catheter 810 which is similar in many respects to catheter 10, wherein like reference numbers indicate similar structure. In this embodiment, the distal tip structure 840 includes a distal tip member 841 similar to that shown in the embodiment of Figure 13, including a ring like distal portion 842, and a proximal portion 844 that includes an elongated tubular section including a plurality of slots and/or grooves 849 cut and/or formed therein where material has been removed from the tubular member. The proximal portion 844 and/or slots 849 can be configured similarly to the proximal portion 744 and/or slots 749 discussed above. However, in this embodiment, the proximal portion 844 extends proximally to the proximal end of the shaft 12, in essence forming at least a portion of what can be characterized as the inner tubular member 22. Such a structure may provide for desirable flexibility characteristics along the length of the shaft, as well as provide for a durable catheter tip. As can be appreciated, other configurations for the distal tip structure 840, such as those discussed above, may be used and may be similarly extended proximally.

In some embodiments, one or more additional layer and/or coating and/or structure may be added to further define the inner tubular member 22. For example, an inner layer or sleeve of material 880 may be added to the inner surface of the structure 840. Such a sleeve 880 may function to provide a tough and/or lubricious and/or low friction coating on the inner surface of the structure 840 such that it is better adapted to function as a guidewire lumen. Additionally, such as layer and/or coating may be used to seal off and/or close the slots and/or grooves 749 such that there is no fluid communication between the guidewire lumen and the inflation lumen. In other embodiments, an outer sleeve and/or layer of material disposed about the structure 840 may be used in combination with and/or instead of using an inner sleeve 880.

Some examples of suitable material that may be used for the sleeve 880 may include polymer materials, such as high-density polyethylene (HDPE), polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b*-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6% LCP.

While a number of structures and configurations have been demonstrated for use in various catheter constructions, those of skill in the art and other will recognize that there are a wide number of variations and combinations of such structures that may be used in the context of this invention. For example, while a number of particular structures for various distal tip members have been shown and discussed, variations and combinations of such structures may be applied to achieve the desired characteristics of the device. Having thus described some embodiments of the invention, those of skill in the art will readily appreciate that yet other embodiments may be made and used within the scope of the claims hereto attached.

## Claims

1. A catheter comprising:
an elongated shaft (12) including distal portion and having a distal end;
a durable polymeric distal tip structure (40) disposed on the distal end of the shaft, the distal tip structure including a distal ring portion (42) configured to enhance the durability of the distal end of the catheter, and a proximal portion (44) extending proximally from the distal ring portion and connecting the distal tip structure to the distal end of the catheter, wherein the proximal portion of the distal tip structure is more laterally flexible than the distal ring portion and
wherein the distal ring portion is made of a polymeric material that is harder than the materials of the distal portion of the elongated shaft, and
wherein at least a portion of the distal tip structure includes a radiopaque material.

2. The catheter of claim 1, wherein the catheter comprises a balloon catheter including an expandable member affixed to the distal portion of the elongated shaft such that a section of the elongated shaft extends through at least a portion of the expandable member.

3. The catheter of any of claims 1-2, wherein the durable distal tip structure comprises a metal or metal alloy.

4. The catheter of any of claims 1-3, wherein the distal ring portion and proximal portion of the durable distal tip structure comprise a single monolith of material.

5. The catheter of any of claims 1-4, wherein the proximal portion comprises a plurality of struts extending proximally from the distal ring portion.

6. The catheter of claim 5, wherein one or more of the plurality of struts includes one or more curved portions.

7. The catheter of claim 5, wherein one or more of the plurality of struts includes a generally straight longitudinal configuration.

8. The catheter of any of claims 1-3, wherein the proximal portion comprises a plurality of struts extending proximally from the distal ring portion, and a proximal ring portion disposed at the proximal end of the struts.

9. The catheter of claim 8, wherein one or more of the plurality of struts includes one or more curved portions.

10. The catheter of any of claims 1-3, wherein the proximal portion comprises a helical structure extending proximally from the distal ring portion.

11. The catheter of any of claims 1-3, wherein the proximal portion comprises a tubular section including a plurality of slots defined therein.

12. The catheter of any of claims 2-11, wherein proximal portion of the distal tip includes a proximal end, and the proximal end of the distal tip structure is distal of the expandable member.

13. The catheter of any of claims 2-11, wherein proximal portion of the distal tip includes a proximal end, and the proximal end of the distal tip structure is proximal of the expandable member.

14. The catheter of any of claims 1-13 wherein the distal tip structure has a length extending from the proximal end to the distal end and wherein the distal ring portion is between 15 and 40% of the length.

15. The catheter of any of claims 1-14 wherein the proximal portion of the distal tip structure is more laterally flexible than the distal portion along its entire length.

## Patentansprüche

1. Katheter, der aufweist:
einen länglichen Schaft (12), der einen distalen Abschnitt aufweist und ein distales Ende aufweist;
eine verschleißfeste polymere distale Spitzenstruktur (40), die am distalen Ende des Schafts angeordnet ist, wobei die distale Spitzenstruktur einen distalen Ringabschnitt (42), der konfiguriert ist, die Verschleißfestigkeit des distalen Endes des Katheters zu verbessern, und einen proximalen Abschnitt (44) aufweist, der sich proximal vom distalen Ringabschnitt erstreckt und die distale Spitzenstruktur mit dem distalen Ende des Katheters verbindet, wobei der proximale Abschnitt der distalen Spitzenstruktur lateral flexibler als der distale Ringabschnitt ist, und
wobei der distale Ringabschnitt aus einem Polymermaterial hergestellt ist, das härter als die Materialien des distalen Abschnitts des länglichen Schafts ist, und
wobei mindestens ein Abschnitt der distalen Spitzenstruktur ein röntgendichtes Material aufweist.

2. Katheter nach Anspruch 1, wobei der Katheter einen Ballonkatheter aufweist, der ein ausdehnbares Element aufweist, das am distalen Abschnitt des länglichen Schafts so befestigt ist, dass sich ein Abschnitt des länglichen Schafts durch mindestens einen Abschnitt des ausdehnbaren Elements erstreckt.

3. Katheter nach einem der Ansprüche 1 bis 2, wobei die verschleißfeste distale Spitzenstruktur ein Metall oder eine Metalllegierung aufweist.

4. Katheter nach einem der Ansprüche 1 bis 3, wobei der distale Ringabschnitt und der proximale Abschnitt der verschleißfesten distalen Spitzenstruktur einen einzelnen Materialmonolith aufweisen.

5. Katheter nach einem der Ansprüche 1 bis 4, wobei der proximale Abschnitt mehrere Streben aufweist, die sich proximal vom distalen Ringabschnitt erstrecken.

6. Katheter nach Anspruch 5, wobei eine oder mehrere der mehreren Streben einen oder mehrere gekrümmte Abschnitte aufweisen.

7. Katheter nach Anspruch 5, wobei eine oder mehrere der mehreren Streben eine im Allgemeinen gerade longitudinale Konfiguration aufweisen.

8. Katheter nach einem der Ansprüche 1 bis 3, wobei der proximale Abschnitt mehrere Streben, die sich proximal vom distalen Ringabschnitt erstrecken, und einen proximalen Ringabschnitt aufweist, der am proximalen Ende der Streben angeordnet ist.

9. Katheter nach Anspruch 8, wobei eine oder mehrere der mehreren Streben einen oder mehrere gekrümmte Abschnitte aufweisen.

10. Katheter nach einem der Ansprüche 1 bis 3, wobei der proximale Abschnitt eine helikale Struktur aufweist, die sich proximal vom distalen Ringabschnitt erstreckt.

11. Katheter nach einem der Ansprüche 1 bis 3, wobei der proximale Abschnitt einen röhrenförmigen Abschnitt aufweist, der mehrere darin definierte Schlitze aufweist.

12. Katheter nach einem der Ansprüche 2 bis 11, wobei der proximale Abschnitt der distalen Spitze ein proximales Ende aufweist und sich das proximale Ende der distalen Spitzenstruktur distal vom ausdehnbaren Element befindet.

13. Katheter nach einem der Ansprüche 2 bis 11, wobei der proximale Abschnitt der distalen Spitze ein proximales Ende aufweist und sich das proximale Ende der distalen Spitzenstruktur proximal vom ausdehnbaren Element befindet.

14. Katheter nach einem der Ansprüche 1 bis 13, wobei die distale Spitzenstruktur eine Länge aufweist, die sich vom proximalen Ende zum distalen Ende erstreckt und wobei der distale Ringabschnitt zwischen 15 und 40% der Länge beträgt.

15. Katheter nach einem der Ansprüche 1 bis 14, wobei der proximale Abschnitt der distalen Spitzenstruktur längs der gesamten Länge lateral flexibler als der distale Abschnitt ist.

## Revendications

1. Cathéter, comprenant :
une tige allongée (12) présentant une partie distale et ayant une extrémité distale ;
une structure polymère durable d'extrémité distale (40) présentée à l'extrémité distale de la tige, ladite structure d'extrémité distale comprenant une partie de bague distale (42) prévue pour accroître la durabilité de l'extrémité distale du cathéter, et une partie proximale (44) s'étendant proximalement depuis la partie de bague distale et reliant la structure d'extrémité distale à l'extrémité distale du cathéter, ladite partie proximale de la structure d'extrémité distale étant plus flexible latéralement que la partie de bague distale, et la partie de bague distale étant constituée d'un matériau polymère plus dur que les matériaux de la partie distale de la tige allongée, et au moins une partie de la structure d'extrémité distale comprenant un matériau radio-opaque.

2. Cathéter selon la revendication 1, où ledit cathéter comprend un cathéter à ballonnet comportant un élément expansible fixé à la partie distale de la tige allongée de sorte qu'une section de la tige allongée s'étend au travers d'au moins une partie de l'élément expansible.

3. Cathéter selon la revendication 1 ou la revendication 2, où la structure durable d'extrémité distale comprend un métal ou un alliage métallique.

4. Cathéter selon l'une des revendications 1 à 3, où la partie de bague distale et la partie proximale de la structure durable d'extrémité distale comprennent un seul monolithe de matériau.

5. Cathéter selon l'une des revendications 1 à 4, où la partie proximale comprend une pluralité d'entretoises s'étendant proximalement depuis la partie de bague distale.

6. Cathéter selon la revendication 5, où une ou plusieurs entretoises de la pluralité comprennent une ou plusieurs parties courbes.

7. Cathéter selon la revendication 5, où une ou plusieurs entretoises de la pluralité présentent une configuration longitudinale sensiblement droite.

8. Cathéter selon l'une des revendications 1 à 3, où la partie proximale comprend une pluralité d'entretoises s'étendant proximalement depuis la partie de bague distale, et une partie annulaire proximale à l'extrémité proximale des entretoises.

9. Cathéter selon la revendication 8, où une ou plusieurs entretoises de la pluralité comprennent une ou plusieurs parties courbes.

10. Cathéter selon l'une des revendications 1 à 3, où la partie proximale comprend une structure hélicoïdale s'étendant proximalement depuis la partie de bague distale.

11. Cathéter selon l'une des revendications 1 à 3, où la partie proximale comprend une section tubulaire où est définie une pluralité de fentes.

12. Cathéter selon l'une des revendications 2 à 11, où la partie proximale de l'extrémité distale comprend une extrémité proximale, et l'extrémité proximale de la structure d'extrémité distale est distale par rapport à l'élément expansible.

13. Cathéter selon l'une des revendications 2 à 11, où la partie proximale de l'extrémité distale comprend une extrémité proximale, et l'extrémité proximale de la structure d'extrémité distale est proximale par rapport à l'élément expansible.

14. Cathéter selon l'une des revendications 1 à 13, où la structure d'extrémité distale a une longueur allant de l'extrémité proximale à l'extrémité distale et où la partie de bague distale représente entre 15 et 40% de ladite longueur.

15. Cathéter selon l'une des revendications 1 à 14, où la partie proximale de la structure d'extrémité distale est plus flexible latéralement que la partie distale sur toute sa longueur.
